# EUROPÄISCHE PATENTANMELDUNG

(11) **EP 3 275 555 A1**
(43) Veröffentlichungstag der Anmeldung: **31.01.2018**
(21) Anmeldenummer: 16181528.7
(22) Anmeldetag: 27.07.2016
(51) Int. Cl.: B05B 11/00, B05B 1/00, B65D 83/20, B65D 83/22, A61M 15/00, A61M 11/00, A61M 11/02, A61M 16/06

(54) **FLÜSSIGKEITSSPENDER, INSBESONDERE INHALATOR**

(71) Anmelder: Aptar Radolfzell GmbH, 78315 Radolfzell (DE)
(72) Erfinder: Lutz, Magnus, 88690 Uhldingen-Mühlhofen (DE); Meinel, Pia, 78467 Konstanz (DE)
(74) Vertreter: Patentanwaltskanzlei Cartagena

(57) **Zusammenfassung**

Bekannt ist ein Flüssigkeitsspender zum Austrag kosmetischer oder pharmazeutischer Flüssigkeiten, insbesondere in Form eines Inhalators. Dieser Flüssigkeitsspender (10) umfasst eine schaltbares Ventileinrichtung (20), eine Basis (40) und einen Austragkopf (60), der zur Betätigung der Ventileinrichtung (20) gegenüber der Basis (40) in einer überlagerten axialen und rotativen Bewegung oder in einer rotativen Bewegung zwischen einer Öffnungsrelativstellung, in der die Ventileinrichtung (20) geöffnet ist, und einer Schließstellung, in derdie Ventileinrichtung (20) geschlossen ist, verlagerbar ist.

Es wird vorgeschlagen, dass in einem Lieferzustand des Flüssigkeitsspenders eine rotative Verlagerung des Austragkopfes (60) gegenüber der Basis (40) durch einen von Spender trennbaren Originalitätsabschnitt (90) gesperrt wird.

## Beschreibung

### ANWENDUNGSGEBIET UND STAND DER TECHNIK

Die Erfindung betrifft einen Flüssigkeitsspender nach dem Oberbegriff von Anspruch 1.

Ein gattungsgemäßer Flüssigkeitsspender ist als mobiler Flüssigkeitsspender ausgebildet, der üblicherweise vom Benutzer bzw. Patienten beispielsweise in einer Handtasche oder Jackentasche mitgeführt werden kann. Insbesondere kann es sich um einen gattungsgemäßen Flüssigkeitsspender handeln, der als Inhalator ausgebildet ist und zu diesem Zweck Flüssigkeit aus einem Flüssigkeitsspeicher des Flüssigkeitsspenders zerstäubt austrägt.

Ein gattungsgemäßer Flüssigkeitsspender verfügt neben einem Flüssigkeitsspeicher über eine Austragvorrichtung, die eine Basis und einen gegenüber der Basis beweglichen Austragkopf umfasst. Eine schaltbare Ventileinrichtung des Flüssigkeitsspenders ist durch die Relativbewegung in einen geöffneten bzw. geschlossenen Zustand überführbar.

Die Relativbeweglichkeit des Austragkopfes gegenüber der Basis kann eine rein rotative Relativbeweglichkeit sein. Sie ist jedoch vorzugsweise eine überlagerte axiale und rotative Relativbeweglichkeit. Insbesondere kann bei einem gattungsgemäßen Spender vorgesehen sein, dass die Relativbeweglichkeit der eines Gewindes entspricht.

### AUFGABE UND LÖSUNG

Aufgabe der Erfindung ist es, für einen gattungsgemäßen Spender eine konstruktiv einfache Möglichkeit zur Verfügung zu stellen, um zu verhindern, dass der Flüssigkeitsspender vor Erstinbetriebnahme versehentlich durch eine Drehbewegung des Austragkopfes gegenüber der Basis aktiviert wird, sei es im Zuge des Handlings vor dem Verkauf des Spenders oder durch den Endkunden.

Erfindungsgemäß wird ein Flüssigkeitsspender zum Austrag kosmetischer oder pharmazeutischer Flüssigkeiten, insbesondere in Form eines Inhalators, vorgeschlagen, der die folgenden Merkmale aufweist.

Der Flüssigkeitsspender umfasst eine schaltbare Ventileinrichtung. Er umfasst eine Basis und einen Austragkopf, der zur Betätigung der Ventileinrichtung gegenüber der Basis in einer überlagerten axialen und rotativen Bewegung oder in einer rotativen Bewegung zwischen einer Öffnungsrelativstellung, in der die Ventileinrichtung geöffnet ist, und einer Schließstellung, in der die Ventileinrichtung geschlossen ist, verlagerbar ist.

In einem Lieferzustand des Flüssigkeitsspenders wird eine rotative Verlagerung des Austragkopfes gegenüber der Basis durch einen von Spender trennbaren Originalitätsabschnitt gesperrt.

Bei einem erfindungsgemäßen Flüssigkeitsspender ist somit vorgesehen, dass dieser Flüssigkeitsspender einen Originalitätsabschnitt aufweist, also einen Abschnitt, der bestimmungsgemäß vor Erstinbetriebnahme entfernt wird, der eine rotative Verlagerung des Austragkopfes gegenüber der Basis verhindert. Erst wenn der Originalitätsabschnitt vom Spender entfernt wurde, ist die Drehbeweglichkeit des Austragkopfes gegenüber der Basis gegeben.

Bei einer Gestaltung eines erfindungsgemäßen Flüssigkeitsspenders mit einer rein rotativen Relativbeweglichkeit des Austragkopfes gegenüber der Basis muss der Originalitätsabschnitt die Drehbewegung unmittelbar verhindern. Er kann hierfür beispielsweise sowohl am Austragkopf als auch an der Basis einstückig angeformt sein, so dass er zumindest von einem dieser Elemente getrennt werden muss, damit die Drehbeweglichkeit gegeben ist. Alternativ kann der Originalitätsabschnitt auch nur am Austragkopf oder an nur der Basis einstückig befestigt sein und mit dem jeweils anderen Element in formschlüssigem Eingriff stehen, bevor ervom Flüssigkeitsspender getrennt wird.

Bei einer Gestaltung, bei der der Austragkopf in einer überlagerten axialen und rotativen Bewegung verlagerbar ist, kann der Originalitätsabschnitt so geartet sein, dass er unmittelbar die axiale Verlagerung des Austragkopfes gegenüber der Basis unterbindet und hierdurch mittelbar auch die rotative Verlagerung verhindert. Dabei ist die axiale Komponente der Relativbeweglichkeit vorzugsweise jene, die unmittelbar durch axiale Verlagerung des Austragkopfes die Ventileinrichtung öffnet oder schließt. Es sind jedoch auch Gestaltungen denkbar, bei denen die Öffnungsrelativstellung und die Schließrelativstellung keine axiale Relativbewegung beim Übergang von der einen Stellung in die andere Stellung erfordern, jedoch zur Inbetriebnahme des Spenders eine Relativbewegung zwischen Austragkopf und Basis in axialer Richtung erforderlich ist.

Insbesondere ein Originalitätsabschnitt, der unmittelbar die Axialbewegung unterbindet, kann als vollständig separates Bauteil vorgesehen sein, welches beispielsweise im Lieferzustand zwischen dem Austragkopf und der Basis derart angeordnet ist, dass diese nicht weiter einander angenähert werden können. Ein solcher separater ringförmiger Qualitätsabschnitt kann während der Montage eingelegt werden und bei Inbetriebnahme als Ganzes entfernt werden, ohne hierbei stofflich von einem der Bauteile des Austragkopfes oder der Basis getrennt zu werden. Von Vorteil ist es jedoch, wenn der Austragkopf ein Gehäuse aufweist, an dem der Qualitätsabschnitt einstückig angeformt ist. Die Verbindung zwischen diesem Gehäuse und dem Qualitätsabschnitt kann beispielsweise in Form dünner bestimmungsgemäß brechender Brücken oder aber in Form eines durchgehenden Verbindungsabschnitts gegeben sein, der über seine gesamte Länge als dünn gestaltete Solltrennstelle ausgebildet ist.

Der Austragkopf ist vorzugsweise zwischen der Schließrelativstellung und Öffnungsrelativstellung zumindest auch axial verlagerbar. Der Originalitätsabschnitt sperrt in Lieferzustand den Austragkopf in der Schließstellung, in der der Austragkopf in einer von der Basis maximal beabstandeten Endlage angeordnet ist.

Der Austragkopf und die Basis sind bei einem erfindungsgemäßen Flüssigkeitsspender üblicherweise aneinander verschieblich geführt und liegen somit stets aneinander an. Unter der genannten maximalen Beabstandung wird verstanden, dass derAustragkopf sich in einer Relativlage gegenüber der Basis befindet, in der er in geringstmöglichen Maße auf die Basis aufgeschoben oder in die Basis eingeschoben ist. Diese maximal beabstandete Endlage geht bei einem erfindungsgemäßen Spender vorzugsweise damit einher, dass die Ventileinrichtung geschlossen ist. Der Originalitätsabschnitt sichert diese maximale Beabstandung und verhindert damit mittelbar ein Öffnen derVentileinrichtung.

Der Originalitätsabschnitt weist vorzugsweise einen Abdeckabschnitt auf, der vor der Trennung ein Sichtfenster des Flüssigkeitsspenders überdeckt, wobei hinter dem Sichtfenster eine Beschriftung angebracht ist.

Gemäß dieser Ausgestaltung übernimmt der Originalitätsabschnitt somit eine Doppelfunktion. Zum einen verhindert er in der bereits beschriebenen Weise eine Relativbewegung zwischen dem Austragkopf und der Basis. Zum anderen stellt er mit dem Abdeckabschnitt einen Abschnitt zur Verfügung, mittels dessen ein Sichtfenster des Flüssigkeitsspenders verschlossen ist. Wird der Originalitätsabschnitt bei der Inbetriebnahme vom Spender getrennt, so wird hierdurch auch das Sichtfenster des Flüssigkeitsspenders geöffnet, so dass auf einen Blick erkennbar ist, dass der Originalitätsabschnitt bereits entfernt wurde.

Wenngleich die Erfindung schwerpunktartig Flüssigkeitsspender betrifft, bei denen zumindest eine rotative Relativbeweglichkeitskomponente bei der Bewegung des Austragkopfes gegenüber der Basis gegeben ist, wird der genannte Aspekt des durch den Originalitätsabschnitt im Lieferzustand abgedeckten Sichtfensters auch bei Flüssigkeitsspendern mit rein axialer Relativbeweglichkeit für vorteilhaft gehalten. Gleiches gilt für die im Weiteren im Kontext des Sichtfensters genannten Aspekte.

Die Beschriftung, die durch das Öffnen des Sichtfensters für den Benutzer lesbar wird, kann im einfachsten Fall eine Beschriftung sein, die auf die erfolgte Inbetriebnahme hinweist, beispielsweise in Form des Hinweises "OPEN". Unter einer Beschriftung sind im Kontext der Erfindung sowohl eine tatsächlich in Buchstaben ausgebildete Beschriftung zu verstehen, jedoch auch Piktogramme oder abgesetzte Farbflächen, die für den Benutzer ähnlich aussagekräftig sein können.

Das Sichtfenster ist vorzugsweise in einem ersten Gehäuseteil vorgesehen, welches gegenüber einem zweiten Gehäuseteil relativbeweglich ist, wobei durch das Sichtfenster hindurch ein Oberflächenabschnitt des zweiten Gehäuseteils sichtbar ist. Die Beschriftung ist am zweiten Gehäuseteil angebracht.

Durch die Anbringung der Beschriftung an einem anderen Gehäuseteil als jenem, in dem das Sichtfenster vorgesehen ist, wird eine fertigungstechnische Vereinfachung ermöglicht. Zudem ist die Beschriftung aufgrund der Relativbeweglichkeit der beiden Gehäuseteile zueinander nicht durchgehend zu sehen, sondern nur in bestimmten Relativstellungen, so dass auch eine die Relativstellung selbst kennzeichnende Beschriftung erzielt werden kann. Dies gilt insbesondere für Ausgestaltungen, bei denen zwei oder mehr Beschriftungen am zweiten Gehäuseteil angebracht sind, so dass aufgrund der Relativbeweglichkeit das Sichtfenster im ersten Gehäuseteil in Abhängigkeit des Betriebszustandes des Flüssigkeitsspenders die eine oder andere Beschriftung zeigt. Entsprechende Beschriftungen könnten beispielsweise "ON" und "OFF" lauten.

Der Abdeckabschnitt bildet vorzugsweise einen Greifabschnitt des Originalitätsabschnitts und weist zu diesem Zweck eine größere Materialstärke als die mittlere Materialstärke des Originalitätsabschnittes und/oder eine radial außenliegende, exponierte Anordnung relativ zu den anderen Teilabschnitten des Qualitätsabschnitts auf.

Dieser Greifabschnitt bildet vorzugsweise ein Ende des insbesondere vorzugsweise etwa bandartigen Originalitätsabschnitts. Da ein solcher Greifabschnitt vorzugsweise ohnehin größer als die übrigen Abschnitte des Originalitätsabschnitts ausgebildet ist, kann durch diesen ein ausreichend großes Sichtfenster am Flüssigkeitsspender im Lieferzustand abgedecktsein.

Durch das dickere Material und/oder durch die Tatsache, dass der Greifabschnitt bezogen auf die Drehachse den am weitest außen gelegenen Teil des Originalitätsabschnitts bildet, ist das Ergreifen des Greifabschnitts vereinfacht. Vorzugsweise sind spezielle Materialbrücken zwischen dem Gehäuse und dem Greifabschnitt vorgesehen, um diesen in Position zu halten, bis der Benutzer den Originatitätsabschnitt entfernt.

Die genannte Relativbeweglichkeit des Austragkopfes und der Basis gegeneinander sind vorzugsweise durch mindestens eine Kulissennut an der Basis oder am Austragkopf sowie einen gewinderartig hierein eingreifenden Steg oder eine entsprechende Nocke gebildet. Vorzugsweise sind mehrere Kulissennuten und hierzu korrespondierend mehrere Stege oder Nocken vorgesehen, um eine besonders sichere Führungzu gewährleisten.

Weiterhin ist es von Vorteil, wenn einer solchen Gestaltung mit mehreren Kulissenbahnen entsprechend die Beschriftungen mehrfach vorgesehen sind. Durch die mehrere Kulissenbahnen ist eine Kopplung der Gehäuseteile im Zuge der Montage in mehreren Stellungen möglich. Durch das mehrfache Vorsehen der Beschriftungen wird erreicht, dass unabhängig von der jeweiligen konkreten Relativstellung der beteiligten Teil bei der Kopplung eine der Beschriftungen im Bereich des Sichtfenster angeordnet ist.

Der Flüssigkeitsspender umfasst üblicherweise einen Flüssigkeitsspeicher, der in Anbetracht der bestimmungsgemäßen Mobilität des Spenders vorzugsweise zur Aufnahme von nicht mehr als 500 ml ausgelegt ist, insbesondere vorzugsweise von nicht mehr als 250 ml. Insbesondere vorzugsweise ist der Flüssigkeitsspeicher als Druckspeicher ausgebildet, also für die Speicherung der Flüssigkeit unter Überdruck vorgesehen, wobei der Überdruck durch komprimiertes Gas, insbesondere Luft, oder durch ein beigefügtes Treibmittel erzielt wird. Auch eine Gestaltung mit einem durch eine Feder kraftbeaufschlagten Speicherkolben ist möglich.

Der Flüssigkeitsspeicher kann insbesondere eines als Inhalator ausgebildeten Flüssigkeitsspenders befüllt sein mit einer der folgenden Flüssigkeiten: Einer salzhaltigen wässrigen Lösung oder einer wässrigen Lösung in Form einer Ringerlösung oder einer gepufferten Lösung oder einer wässrigen Lösung mit mindestens einem der Zusätze Kohlenhydrate, ätherische Öle, Menthol und Pflanzenextrakte oder einer wässrigen Lösung, enthaltend Vitamine, Spurenelemente, Mangan oder Zink, oder einer wässrigen Lösung mit mindestens einem der Zusätze aus der Gruppe umfassend Zimtöl, Teebaumöl, Salbeiöl, Thymianöl, Zitronenmelissenöl.

Der Flüssigkeitsspender ist als Inhalator ausgebildet und verfügt über ein Atemstück, welches als Mundstück oder als Atemmaske ausgestaltet ist.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert ist.
Fig. 1 und Fig. 2 zeigen einen erfindungsgemäßen Flüssigkeitsspender in einer Gesamtdarstellung wobei der Flüssigkeitsspeicher in Fig. 2 geschnitten dargestellt ist.
Fig. 3 zeigt ein Gehäuseteil der Basis sowie ein Gehäuseteil des Austragkopfes mitsamt am Austragkopf angebrachtem Originalitätsabschnitt.
Fig. 4 zeigt die Gehäuseteile der Fig. 3 in ihrer Relativstellung im Lieferzustand.
Fig. 5 und 6 zeigen die beiden Relativendlagen der Gehäuseteile der Fig. 3 und 4 nach Entfernen des Originalitätsabschnittes.

### DETAILLIERTE BESCHREIBUNG DERAUSFÜHRUNGSBEISPIELE

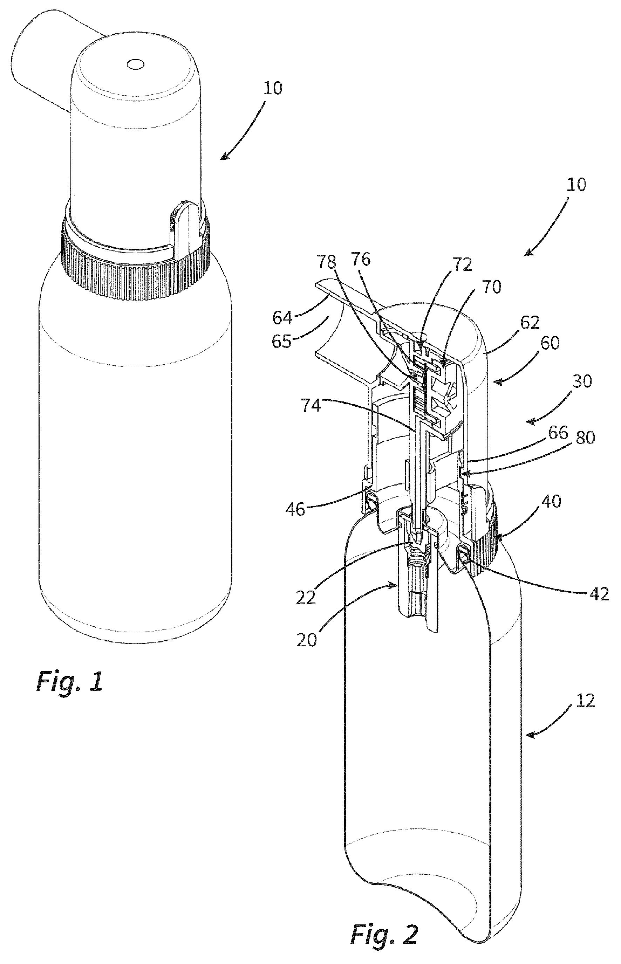

Die Fig. 1 und 2 zeigen eine Ausführungsform eines erfindungsgemäßen Spenders in einer Gesamtdarstellung sowie einer geschnittenen Darstellung. Der erfindungsgemäße Spender 10 verfügt über einen als Druckspeicher ausgebildeten Flüssigkeitsspeicher 12 sowie einen Austragkopf 30. Der Austragkopf 30 umfasst eine Basis 40, welches mittels einer Schnappverbindung 42 mit dem Flüssigkeitsspeicher 12 verbunden ist und gegenüber dem Flüssigkeitsspeicher 12 nicht drehbar ist.

Der Spender umfasst weiterhin einen Austragkopf 60, welcher in im Weiteren noch erläuterter Weise relativbeweglich gegenüber der Basis 40 ist, um hierdurch den Flüssigkeitsaustragzu steuern.

Der Austragkopf 60 verfügt über ein Außengehäuse 62, in welchem zwei Innenbauteile 70, 72 befestigt sind, wobei das Innenbauteil 72 ein Zuführrohr 74 umfasst, welches sich bis in eine am Flüssigkeitsspeicher 12 vorgesehene Ventileinrichtung 20 erstreckt und dort auf einem axial verlagerbaren Ventilkörper 22 aufliegt.

Bei einer Relativbewegung des Austragkopfes 60 gegenüber der Basis 40, im Zuge derer das Zuführrohr 74 in Richtung des Flüssigkeitsspeichers verlagert wird, strömt Flüssigkeit aus dem Flüssigkeitsspeicher durch das Zuführrohr 74 sowie durch eine Filtereinheit 76 bis zu einer Düseneinheit 78, die in nicht näher dargestellter Weise eine Vielzahl von Einzeldüsenöffnungen umfasst. Hier wird die Flüssigkeit vernebelt und gelangt in einen Austrittskanal 65 eines Atemstückes 64, welches Teil des Außengehäuses 62 ist.

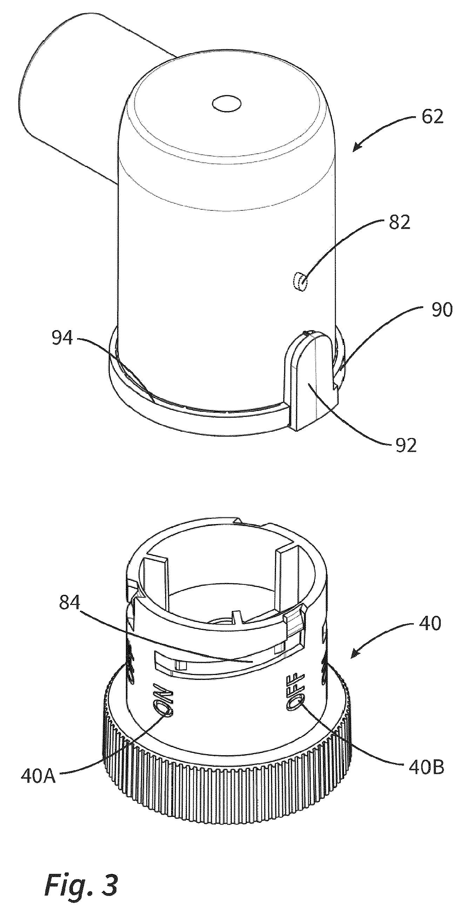

Die Basis 40 und das Außengehäuse 62 des Austragkopfes 60 definieren gemeinsam die Relativbeweglichkeit des Austragkopfes 60 gegenüber der Basis 40 und somit gegenüber dem Flüssigkeitsspeicher 12. Wie aus Fig. 3 ersichtlich ist, ist im Basisbauteil 40 eine Kulissennut 84 vorgesehen. Korrespondierend hierzu ist an der Innenseite eines Hülsenabschnitts 66 des Außengehäuses 62 eine Gewindenocke 82 angebracht, die in die Kulissennut 84 hineinragt. Da die Kulissennut in der aus Fig. 3 ersichtlichen Weise eine Helixabschnittsform aufweist, ist der Austragkopf 60 in einer entsprechend überlagerten relativen Axial- und Rotativbewegung gegenüber der Basis verlagerbar. Durch eine Drehbewegung des Austragkopfes 60 gegenüber der Basis 40 wird somit mittelbar eine axiale Verlagerung bewirkt, die geeignet ist, die Ventileinrichtung 20 zu öffnen bzw. zu schließen.

Aus Fig. 3 ist darüber hinaus ersichtlich, dass umlaufend am unteren offenen Ende des Außengehäuses 62 ein Originalitätsabschnitt 90 vorgesehen ist. Es handelt sich um ein umlaufendes Originalitätsband, an dessen Ende ein Greifabschnitt vorgesehen ist, der als Abdeckabschnitt 92 agiert. Der Originalitätsabschnitt 90 mitsamt dem Greifabschnitt 92 ist durch Materialbrücken 94 mit dem Außengehäuse 62 stoffschlüssig verbunden.

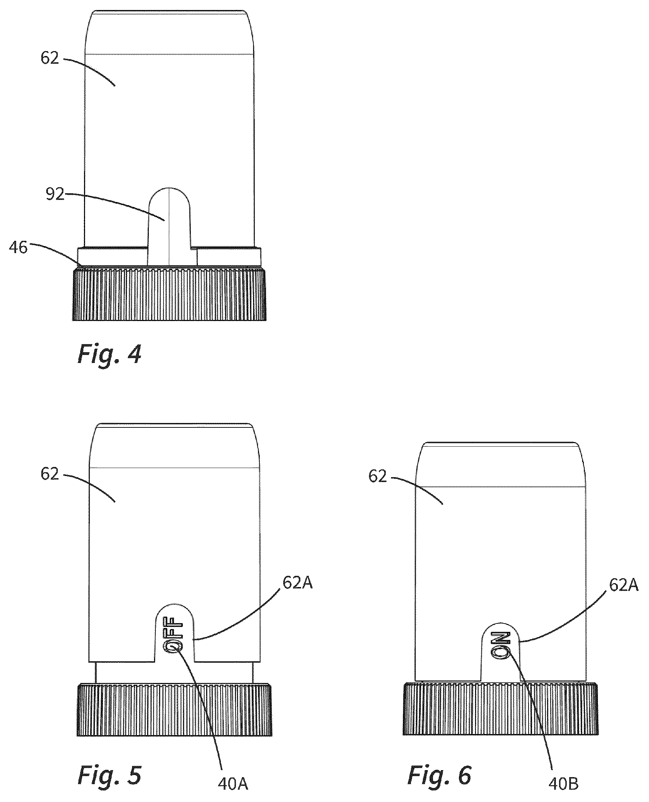

Bezug nehmend auf Fig. 4 ist ersichtlich, dass eine axiale Verlagerung des Außengehäuses 62 gegenüber der Basis 40 bei noch angebrachtem Originalitätsabschnitt 90 nicht möglich ist. Der Originatitätsabschnitt würde bei einer solchen Bewegung mit einer Stufe 46 der Basis 40 kollidieren. Da eine axiale Verlagerung nicht möglich ist, ist auch eine überlagerte rotative und axiale Verlagerung bei angebrachtem Originalitätsabschnitt nicht möglich. Der Originalitätsabschnitt muss zunächst entfernt werden, so dass sich der Zustand der Fig. 5 einstellt.

Wenn dies gegeben ist, kann anschließend die zur Aktivierung des Spenders vorgesehene überlagerte axiale und rotative Bewegung stattfinden und somit ein Flüssigkeitsaustrag bewirkt werden.

Wie die Fig. 5 und 6 zeigen, wird durch Entfernen des Originalitätsabschnitts 90 und insbesondere des Abdeckabschnitt 92 ein Sichtfenster 62A geöffnet, welches eine darunterliegende Beschriftung sichtbar macht. Wie anhand der Fig. 3 ersichtlich ist, sind zwei solche Beschriftungen an der Außenseite des der Basis 40 vorgesehen, so dass in Abhängigkeit der Relativstellung des Außengehäuses 62 im Fenster unmittelbar zu erkennen ist, ob der Spender aktiviert oder deaktiviert ist.

## Patentansprüche

1. Flüssigkeitsspender (10) zum Austrag kosmetischer oder pharmazeutischer Flüssigkeiten, insbesondere in Form eines Inhalators, mit den folgenden Merkmalen:
a. der Flüssigkeitsspender (10) umfasst ein schaltbare Ventileinrichtung (20) und
b. der Flüssigkeitsspender (10) umfasst eine Basis (40) und
c. der Flüssigkeitsspender (10) umfasst einen Austragkopf (60), der zur Betätigung derVentileinrichtung (20) gegenüber der Basis (40) in einer überlagerten axialen und rotativen Bewegung oder in einer rotativen Bewegung zwischen einer Öffnungsrelativstellung, in der die Ventileinrichtung (20) geöffnet ist, und einer Schließstellung, in der die Ventileinrichtung (20) geschlossen ist, verlagerbar ist,
**gekennzeichnet durch** das Merkmal:
d. in einem Lieferzustand des Flüssigkeitsspenders (10) wird eine rotative Verlagerung des Austragkopfes (60) gegenüber der Basis (40) **durch** einen von Flüssigkeitsspender trennbaren Originalitätsabschnitt (90) gesperrt.

2. Flüssigkeitsspender (10) nach Anspruch 1 mit den folgenden Merkmalen:
a. der Austragkopf (60) ist in einer überlagerten axialen und rotativen Bewegung verlagerbar, und
b. der vom Flüssigkeitsspender (10) trennbare Originalitätsabschnitt (90) sperrt im Lieferzustand des Flüssigkeitsspenders (10) die axiale Verlagerung des Austragkopfes (60) gegenüber der Basis (40) und dadurch mittelbar die rotative Verlagerung.

3. Flüssigkeitsspender (10) nach Anspruch 1 oder 2 mit den folgenden Merkmalen:
a. der Austragkopf (60) weist ein Gehäuseteil (62) auf und
b. der trennbare Originalitätsabschnitt (90) ist ein einstückig mit einem Abschnitt des Gehäuseteils (62) verbundenerAbschnitt.

4. Flüssigkeitsspender (10) einem der vorstehenden Ansprüchen mit dem folgenden Merkmal:
a. der trennbare Originalitätsabschnitt (90) ist ein ringförmiger Abschnitt, der den Flüssigkeitsspender (10) mindestens um 180° umgibt, vorzugsweise mindestens um 300°, insbesondere vorzugsweise vollständig.

5. Flüssigkeitsspender (10) nach einem der vorstehenden Ansprüche mit den folgenden Merkmalen:
a. der Austragkopf (60) ist zwischen der Schließrelativstellung und Öffnungsrelativstellung axial verlagerbar, und
b. der Originalitätsabschnitt (90) sperrt im Lieferzustand den Austragkopf (60) in der Schließstellung, in der der Austragkopf (60) in einer von der Basis (40) maximal beabstandeten Endlage angeordnet ist.

6. Flüssigkeitsspender (10) nach einem der vorstehenden Ansprüche mit dem folgenden Merkmal:
a. der Originalitätsabschnitt (90) weist einen Abdeckabschnitt (92) auf, der vor der Trennung ein Sichtfenster (62a) des Flüssigkeitsspenders (10) überdeckt, wobei hinter dem Sichtfenster (62a) eine Beschriftung (40A, 40B) angebracht ist.

7. Flüssigkeitsspender (10) nach Anspruch 6 mit den folgenden Merkmalen:
a. das Sichtfenster (62a) ist in einem ersten Gehäuseteil (62) vorgesehen, welches gegenüber einem zweiten Gehäuseteil (40) relativbeweglich ist, wobei durch das Sichtfenster (62a) hindurch ein Oberflächenabschnitt des zweite Gehäuseteils (40) sichtbar ist, und
b. die Beschriftung (40A, 40B) ist am zweiten Gehäuseteil (40) angebracht.

8. Flüssigkeitsspender (10) nach Anspruch 7 mit dem folgenden Merkmal:
a. am zweiten Gehäuseteil (40) sind mindestens zwei Beschriftungen (40A, 40A) angebracht, die in Abhängigkeit der Relativstellung des ersten Gehäuseteils (62) gegenüber dem zweiten Gehäuseteil (40) durch das Sichtfenster (62A) hindurch sichtbar oder durch das erste Gehäuseteile (62) verdeckt sind.

9. Flüssigkeitsspender (10) nach einem der Ansprüche 6 bis 8 mit dem folgenden Merkmal:
a. der Abdeckabschnitt (92) bildet einen Greifabschnitt des Originalitätsabschnitts (90) und weist zu diesem Zweck eine größere Materialstärke als die mittlere Materialstärke des Originalitätsabschnittes (90) und/oder eine radial außenliegende, exponierte Anordnung relativ zu den anderen Teilabschnitten des Qualitätsabschnitts (90) auf.

10. Flüssigkeitsspender (10) nach einem der vorstehenden Ansprüche mit mindestens einem der folgenden Merkmale:
a. die Basis (40) und derAustragkopf (60) sind mittels einer Kulissennut (84), in der ein Kulissengleiter (88) angeordnet ist, gegeneinander in einer überlagerten axialen und rotativen Bewegung verlagerbar und/oder
b. der Flüssigkeitsspender (10) umfasst einen Flüssigkeitsspeicher (12) zur Lagerung von Flüssigkeit vor dem Versprühen, der vorzugsweise als Druckspeicher ausgebildet ist, und/oder
c. der Flüssigkeitsspeicher (12) ist zur Aufnahme von maximal 500 ml ausgelegt, vorzugsweise von maximal 250 ml und/oder
d. der Flüssigkeitsspeicher (12) ist befüllt mit:
- einer salzhaltigen wässrigen Lösung oder
- einer wässrigen Lösung in Form einer Ringerlösung oder einer gepufferten Lösung oder einer wässrigen Lösung mit mindestens einem der Zusätze Kohlenhydrate, ätherische Öle, Menthol und Pflanzenextrakte oder
- einer wässrigen Lösung, enthaltend Vitamine, Spurenelemente, Mangan oder Zink, oder
- einer wässrigen Lösung mit mindestens einem der Zusätze aus der Gruppe umfassend Zimtöl, Teebaumöl, Salbeiöl, Thymianöl, Zitronenmelissenöl und/oder
e. der Flüssigkeitsspender (10) ist als Inhalator ausgebildet und verfügt über ein Atemstück (64), welches als Mundstück oder als Atemmaske ausgestaltet ist, und/oder
f. das Atemstück (64) weist einen Kanalabschnitt auf, der in einer von einer Betätigungsrichtung des Austragskopfs abweichenden Richtung ausgerichtet ist und vorzugsweise mit dieser einen Winkel zwischen 45° und 135° einschließt, insbesondere vorzugsweise einen Winkel zwischen 70° und 110°.
